# EUROPEAN PATENT APPLICATION

(11) **EP 2 960 331 A1**
(43) Date of publication of application: **30.12.2015**
(21) Application number: 14754539.6
(22) Date of filing: 20.02.2014
(51) Int. Cl.: C12N 15/09, A01H 1/00, A01H 5/00, C12N 5/10

(54) **NUCLEIC ACID MOLECULE FOR EXPRESSING FOREIGN GENE IN PLANT AND METHOD THEREFOR**

(30) Priority: 20.02.2013 JP 2013031355
(71) Applicant: National Institute of Advanced Industrial Science and Technology, Tokyo 100-8921 (JP); National University Corporation Hokkaido University, Sapporo-shi, Hokkaido 060-0808 (JP)
(72) Inventor: MATSUMURA, Takeshi, Sapporo-shi Hokkaido 062-8517 (JP); FUKUZAWA, Noriho, Sapporo-shi Hokkaido 062-8517 (JP); ITCHOUDA, Noriko, Sapporo-shi Hokkaido 062-8517 (JP); MASUTA, Chikara, Sapporo-shi Hokkaido 060-0808 (JP)
(74) Representative: Tischner, Oliver
(86) International application number: PCT/JP2014/054083
(87) International publication number: WO 2014/129560

(57) **Abstract**

A nucleic acid molecule for expressing a foreign gene in a plant, said nucleic acid molecule carrying, in this order, a right border (RB) sequence derived from the T-DNA sequence of an agrobacterium, an expression promoter sequence capable of functioning in the aforesaid plant, a sequence corresponding to the RNA2 genome of cucumber mosaic virus wherein a 2b protein-encoding gene has been partly or entirely substituted by the aforesaid foreign gene, and a left border (LB) sequence derived from the T-DNA sequence of an agrobacterium.

## Description

### TECHNICAL FIELD

The present invention relates to a nucleic acid molecule and a method for expressing a foreign gene in a plant.

### BACKGROUND ART

Transient expression technique, which can express a target gene in a plant in about one to two weeks, is an effective means for producing a substance using a plant.

Conventional transient expression methods using plants are generally categorized into (1) agroinfiltration method, (2) plant virus vector method, (3) MagnICON^{®} system, and (4) particle gun method.

(1) The agroinfiltration method involves infecting a plant with *Agrobacterium* transformed with a T-DNA vector into which a target gene has been inserted, e.g., by introducing a culture of the transformed *Agrobacterium* into tissues of the plant via physical means (such as syringe injection or permeation under reduced pressure), such that the target gene is transiently expressed in the plant (Non-Patent Document 1: Grimsley N., Hohn B., Hohn T., and Walden R., (1986), "Agroinfection," an alternative route for viral infection of plants by using the Ti plasmid an alternative route for viral infection of plants by using the Ti Plasmid., Proc Natl Acad Sci USA, 83(10):3282-6). Since *Agrobacterium* with strong infectability is transferred throughout a plant via physical means (such as injection or permeation) and caused to infect the plant, the target gene can be expressed evenly in all plant tissues. However, the expression level of the target gene at each cell is limited by the infectability of *Agrobacterium* and the capabilities of the expression modulation sequences (e.g., the promoter and the terminator) in the introduced gene. Therefore, it is difficult to improve the total expression level in a plant.
(2) The plant virus vector method involves in-vitro transcribing cDNA of the genome of a plant virus into which a target gene has been inserted, and inoculating the resultant RNA as a vector into a plant to cause infection, such that the target gene is expressed in the plant based on the amplification ability and the systemic transition ability of the virus. Examples of such plant virus vectors include: cucumber mosaic virus (CMV) vector, developed by the present inventors (Patent Document 1: JP4009663B); tobacco mosaic virus (TMV) vector (Non-Patent Document 2: Takamatsu N, Ishikawa M, Meshi T, and Okada Y., (1987), Expression of bacterial chloramphenicol acetyltransferase gene in tobacco plants mediated by TMV-RNA, EMBO J., 6(2):307-11); and potato X virus (PVX) vector (Non-Patent Document 3: Baulcombe, D.C., Chapman, S., and Santa Cruz, S., (1995), Jellyfish green fluorescent protein as a reporter for virus infectious, Plant J., 7:1045-1053). Since this method employs the autonomous replication ability of a virus for causing expression of a target gene, it is possible to increase the expression level of the target gene per cell in plant by using a vector having strong amplification ability, such as a CMV- or TMV-based vector. However, it is very difficult to cause infection of the virus vector and expression of the target gene in all cells throughout the plant, since transition of a vector into each tissue cell of the plant depends on the transition ability of the virus. As a result, the plant resulting from introduction of the vector is in a chimeric (mosaic) state composed of a first population of cells, which are infected with the virus and express the target gene, and a second population of cells, which are not infected with the virus and does not express the target gene. Therefore, it is also difficult to improve the total expression level in a plant.
(3) The MagnICON^{®} system is prepared by incorporating, into T-DNA vector, cDNA of the TMV or PVX genome in which a target gene has been inserted. *Agrobacterium* is transformed with the resultant vector, and a culture of the transformed *Agrobacterium* is introduced into tissues of a plant via physical means (such as syringe injection or permeation under reduced pressure), such that the target gene is transiently expressed in the plant (Patent Document 2: JP2007-510423A; Patent Document 3: JP2008-535473A; and Patent Document 4: JP2011-024591A). According to this system, it is possible to systemically introduce the vector throughout the plant via physical means (such as injection or permeation) to cause infection, thereby allowing for expression of the target gene in all tissues of the plant. In addition, since this system employs the autonomous replication ability of a virus for causing expression of the target gene, it is possible to increase the expression level of the target gene per cell in plant. Thus, this system combines advantages of both (1) the agroinfiltration method and (2) the plant virus vector method. However, since this system can only be established on a limited type of plant viruses whose genome is composed of one single-stranded RNA having high amplification ability, such as TMV or PVX, the host plant to be infected by this vector system is quite limited. In addition, since all of the genes necessary for replication of the virus (TMV or PVX) have to be arranged within the one single-stranded RNA in addition to the target gene to be expressed, the size of the target gene to be inserted is also quite limited.
(4) The particle gun (particle bombardment) method involves injecting metal microparticles coated with nucleic acids to a cell at a high speed to thereby introduce a target gene into a cell (Non-Patent Document 4: Christou P, (1995), Particle bombardment, Methods Cell Biol., 50:375-82). Although it can easily be carried out using various organisms or tissues, this method is not practical due to various problems such as the relatively high cost involved in this method, the possibility that the target gene may be disrupted on introduction, and the extremely low introduction rate into cells in plant.

### PRIOR ART REFERENCES

### PATENT DOCUMENTS

[Patent Document 1] JP4009663B
[Patent Document 2] JP2007-510423A
[Patent Document 3] JP2008-535473A
[Patent Document 4] JP2011-024591A

### NON-PATENT DOCUMENTS

[Non-Patent Document 1] Grimsley N., Hohn B., Hohn T., and Walden R., (1986), "Agroinfection," an alternative route for viral infection of plants by using the Ti plasmid an alternative route for viral infection of plants by using the Ti Plasmid., Proc Natl Acad Sci USA, 83(10):3282-6
[Non-Patent Document 2] Takamatsu N, Ishikawa M, Meshi T, and Okada Y., (1987), Expression of bacterial chloramphenicol acetyltransferase gene in tobacco plants mediated by TMV-RNA, EMBO J., 6(2):307-11
[Non-Patent Document 3] Baulcombe, D.C., Chapman, S., and Santa Cruz, S., (1995), Jellyfish green fluorescent protein as a reporter for virus infectious, Plant J., 7:1045-1053
[Non-Patent Document 4] Christou P, (1995), Particle bombardment, Methods Cell Biol., 50:375-82

### DISCLOSURE OF THE INVENTION

### PROBLEMS TO BE SOLVED BY THE INVENTION

Under these circumstances, there is a demand for an excellent transient expression method which exhibits superior systemic transition ability into a plant and causes high level expression of the target gene in most cells in the plant, while involving less restrictions to the type of host plant to be used or the size of target gene to be inserted.

### MEANS TO SOLVE THE PROBLEMS

After intensive investigations in view of the aforementioned problems, the present inventors have finally reached the following method. A sequence corresponding to the RNA2 genome of cucumber mosaic virus (CMV), of which the gene coding for 2b protein has been partly or entirely replaced with a foreign gene, is operably linked to an Agrobacterium T-DNA sequence to construct a nucleic acid molecule, which is then introduced into a host plant which effectively expresses the RNA1 genome and the RNA3 genome of the CMV as well as the protein 2b of the CMV, and the resultant plant is cultured to cause expression of the foreign gene. This method allows for systemic transition of the foreign gene into cells throughout the plant, thereby improving the expression efficiency of the foreign gene in each cell to achieve increased expression of the foreign gene in the plant as a whole. This method also expands the freedom of choice for the type of host plant to be used and the size of target gene to be inserted, compared with the MagnICON^{®} system based on TMV or PVX. Thus, the present inventors have arrived at the present invention.

Specifically, aspects of the present invention include the following:
(1) A nucleic acid molecule for expressing a foreign gene in a plant, comprising, arranged in this order: a right border sequence (RB) derived from the *Agrobacterium* T-DNA sequence; an expression promoter sequence workable in the plant; a sequence corresponding to the RNA2 genome of cucumber mosaic virus in which a part or all of the gene coding for the 2b protein has been replaced with the foreign gene; and a left border sequence (LB) derived from the *Agrobacterium* T-DNA sequence.
(2) The nucleic acid molecule according to (1), further comprising a terminator sequence workable in the plant, arranged between the sequence corresponding to the RNA2 genome of cucumber mosaic virus and the left border sequence (LB) derived from Agrobacterium T-DNA sequence.
(3) The nucleic acid molecule according to (1) or (2), which is in the form of a T-DNA vector.
(4) The nucleic acid molecule according to any one of (1) to (3), wherein the plant functionally expresses the RNA1 genome of cucumber mosaic virus, the RNA3 genome of cucumber mosaic virus, and the 2b protein of cucumber mosaic virus.
(5) A method for expressing a foreign gene in a plant, comprising: functionally expressing the RNA1 genome of cucumber mosaic virus, the RNA3 genome of cucumber mosaic virus, and the 2b protein of cucumber mosaic virus in the plant; introducing the nucleic acid molecule according to any one of (1) to (3) into the plant; and culturing the plant containing the nucleic acid molecule to thereby express the foreign gene incorporated in the nucleic acid molecule in the plant.
(6) The method according to (5), wherein expression of the RNA1 genome of cucumber mosaic virus and the RNA3 genome of cucumber mosaic virus is carried out by introducing, into the plant, a nucleic acid molecule comprising, arranged in this order, a right border sequence (RB) derived from *Agrobacterium* T-DNA sequence, an expression promoter sequence workable in the plant, a sequence corresponding to the RNA1 genome of cucumber mosaic virus, and a left border sequence (LB) derived from *Agrobacterium* T-DNA sequence; and a nucleic acid molecule comprising, arranged in this order, a right border sequence (RB) derived from *Agrobacterium* T-DNA sequence, an expression promoter sequence workable in the plant, a sequence corresponding to the RNA3 genome of cucumber mosaic virus, and a left border sequence (LB) derived from *Agrobacterium* T-DNA sequence.
(7) The method according to (5), wherein the introduction of the nucleic acid molecules into the plant is carried out by infiltrating or injecting a solution containing the nucleic acid molecules into the plant.

### EFFECTS OF THE INVENTION

The present invention allows for systemic transition of a foreign gene into the cells throughout the plant and systemic expression of the foreign gene with increasing expression efficiency of the foreign gene from each cell, thereby achieving high level expression of the plant as a whole. The present invention also allows for transient expression using a variety of host plants and a variety of foreign genes different in size.

### BRIEF EXPLANATION OF THE DRAWINGS

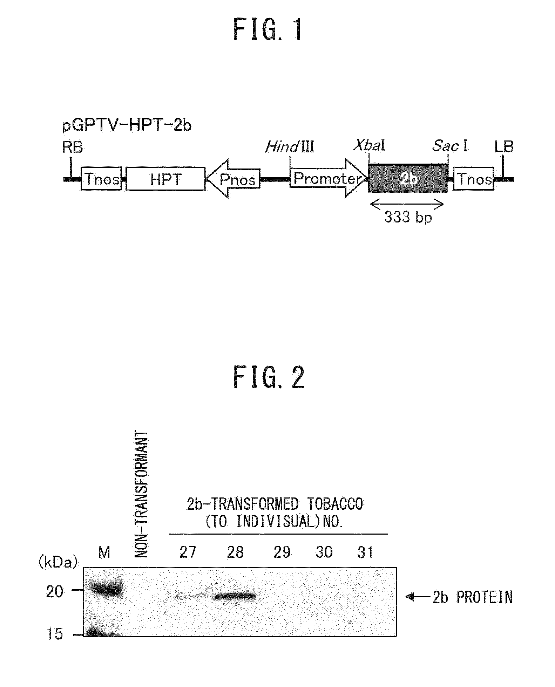

[Fig. 1] Fig. 1 schematically illustrates the construction of a CMV-2b gene plant expression vector (pGPTV-HPT-2b: nucleic acid molecule B), in which "HPT" represents a hygromycin resistance gene, "Tnos" represents a Nos terminator, "Pnos" represents a Nos promoter, "Promoter" represents an internal fragment sequence derived from a strawberry vein banding virus (SVBV) promoter + a 5'-upstream region (Ω region) of the tobacco mosaic virus (TMV), and "2b" represents a gene encoding the CMV-2b protein.
[Fig. 2] Fig. 2 is a photograph indicating the results of western blotting analysis showing the expression of the 2b protein from a 2b-transformed *Nicotiana benthamiana* tobacco plant.

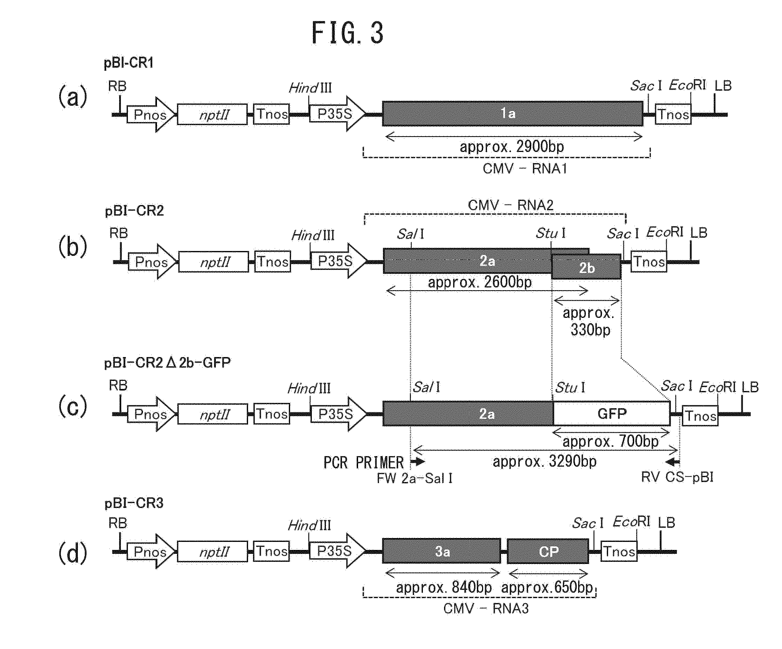

[Fig. 3] Figs. 3(a) to (d) schematically illustrate the construction of a binary vector for plant expression. Specifically, Fig. 3(a) illustrates a vector containing CMV genome 1 (nucleic acid molecule A), Fig. 3(b) illustrates a vector containing CMV genome 2, Fig. 3(c) illustrates a vector containing CMV genome 2 in which a 2b gene region has been replaced with a green fluorescent protein (GFP) as a target gene (a nucleic acid molecule of the present invention), and Fig. 3(d) illustrates a vector containing CMV genome 3 (nucleic acid molecule C). "Pnos" represents a Nos promoter, "nptII" represents a kanamycin resistance gene, "Tnos" represents a Nos terminator, "P35S" represents a promoter sequence derived from the cauliflower mosaic virus (CaMV), "FW-2a-Sal" with an arrow and "RV CS-pBI" with an arrow represent PCR primer sites used for inserting the green fluorescent protein (GFP) being the target gene.

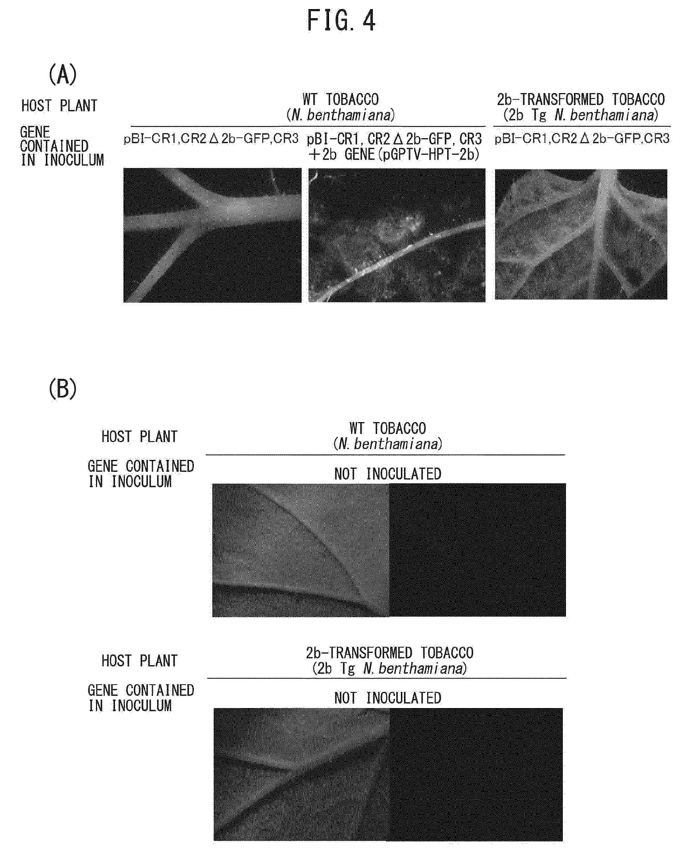

[Fig. 4] Figs. 4(A) and (B) are photographs of fluorescent microscopic images of leaves of the inoculated plant. Specifically, Fig. 4(A) indicates fluorescent micrographs of leaves of a wild-type plant and a 2b-transformed plant taken via GFP detection after agroinfiltration (five days after inoculation), in which the autofluorescent wavelength from leaves was filtered off. Bright sections (green on the original photos) correspond to regions expressing GFP, while black sections correspond to leave regions without luminescence from GFP. Fig. 4(B) indicates fluorescent micrographs of leaves of a non-inoculated plant (negative control). Bright sections on the light photograph (red on the original photo) indicate autofluorescence from leaves, while right photograph is the same as the light photograph except that the autofluorescent wavelength leaves from leaves was filtered off. Green luminescence from GFP was not detected.

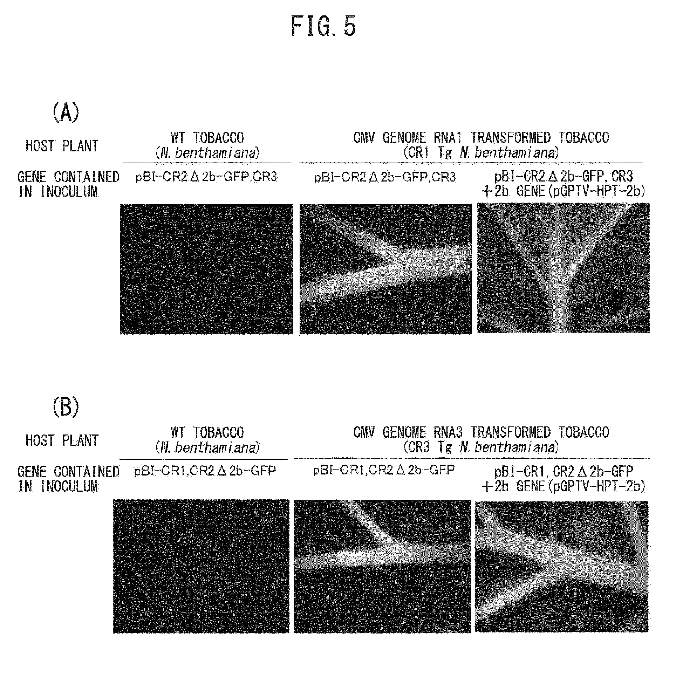

[Fig. 5] Figs. 5(A) and (B) are photographs of fluorescent microscopic images of leaves inoculated with transformants containing parts of the CMV genome. Specifically, Fig. 5(A) indicates micrographs of leaves of a tobacco transformed with CMV genome RNA1 (CR1-Tg) taken via GFP detection after agroinfiltration (seven days after inoculation). Fig. 5(B) indicates micrographs of leaves of a tobacco transformed with CMV genome RNA3 (CR3-Tg) taken via GFP detection after agroinfiltration (five days after inoculation). Bright sections (green on the original photos) correspond to regions expressing GFP, while black sections correspond to regions without luminescence from GFP.

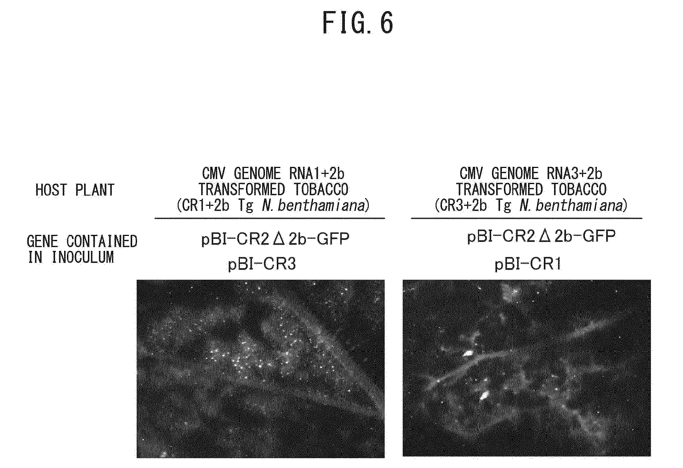

[Fig. 6] Fig. 6 indicates fluorescent micrographs of leaves inoculated with a transformant containing the 2b gene and a part of the CMV genome. The left photograph is a micrograph of leaves of a tobacco transformed with the 2b gene and CMV genome RNA1 (CR1+2bTg) taken via GFP detection after agroinfiltration (seven days after inoculation). The right photograph is a micrograph of leaves of a tobacco transformed with the 2b gene and CMV genome RNA3 (CR3+2bTg) taken via GFP detection after agroinfiltration (five days after inoculation). Bright sections (green on the original photos) correspond to regions expressing GFP, while black sections correspond to regions without luminescence from GFP.

### MODE FOR CARRYING OUT THE INVENTION

The present invention will be explained in detail below by reference to specific embodiments. It should be noted, however, that the present invention should not be limited to the following embodiments, but can be worked with making modifications thereto as appropriate.

### [1. Introduction]

An explanation is first provided of the Agrobacterium and cucumber mosaic virus on which the present invention is premised.

### [1-1. Agrobacterium]

Agrobacterium is the generic name for Gram-negative soil bacteria belonging to the genus Rhizobium that demonstrate pathogenicity against plants. One example of Agrobacterium is Agrobacterium tumefaciens that is associated with crown gall.

Agrobacterium possesses a giant plasmid referred to as tumor-inducing (Ti) plasmid. The Ti plasmid has a domain referred to as a transfer DNA (T-DNA) sequence, and has the property of being incorporated in a plant genome by random insertion as a result of a DNA fragment corresponding to that domain migrating to a cell in plant. By utilizing this property, Ti plasmid can be used for the purpose of, for example, introducing and expressing a foreign gene in a plant by transformation.

The T-DNA sequence has a right border sequence (to be abbreviated as "RB sequence" or simply "RB") and a left border sequence (to be abbreviated as "LB sequence" or simply "LB") on both ends thereof that are required for incorporation into a genome, and has plant hormone biosynthesis genes and opine biosynthesis genes between the RB sequence and LB sequence.

Since Ti plasmid contains sequences essential for plant transformation, including the aforementioned plant hormone biosynthesis genes and opine biosynthesis genes, it is quite large, resulting in poor manipulability. Accordingly, introduction of a foreign gene by plant transformation is currently primarily carried out using a T-DNA binary system combining two types of modified Ti plasmids, namely a binary plasmid and a helper plasmid.

The binary plasmid serves as a shuttle vector enabling replication of both Agrobacterium and other microorganisms by incorporating a sequence from which domains not required for plant transformation have been removed from the T-DNA domain (such as the domains of the aforementioned plant hormone biosynthesis genes and opine biosynthesis genes), and a replication origin capable of functioning in both Agrobacterium and other microorganisms (such as Escherichia coli). The binary plasmid normally does not have the entire T-DNA sequence, but rather only has an RB sequence and LB sequence (along with their neighboring domains) derived from the T-DNA sequence, and optionally has a nos promoter and nos terminator, which regulate expression of removed genes such as plant hormone biosynthesis genes and opine biosynthesis genes, between the RB sequence and LB sequence. The foreign gene to be introduced into a plant is arranged between the RB sequence and LB sequence and optionally arranged downstream from the aforementioned nos promoter and nos terminator. In general, a "T-DNA vector" refers to this binary plasmid.

The helper plasmid refers to a plasmid that has been reduced in size by removing the T-DNA sequence and other domains not required for host recombination and so forth of the T-DNA sequence from a Ti plasmid while leaving behind a gene cluster in the form of a vir domain that induces random incorporation of the T-DNA sequence into a genome. The function of the vir domain of inducing recombination of the T-DNA domain into a genome is known to be trans-acting. Accordingly, even if the aforementioned T-DNA vector does not have a vir domain, by incorporating this helper plasmid having a vir domain into Agrobacterium together with the T-DNA vector, the T-DNA domain present in the T-DNA vector can be induced to be incorporated into a genome, and in turn, a foreign gene arranged between the RB sequence and LB sequence can be expressed by incorporating into a plant genome.

Furthermore, a vir domain can be incorporated in the aforementioned T-DNA vector and be made to function as an independent vector without using this type of helper plasmid having a vir domain.

In the case of transiently expressing a foreign gene in a plant by using such a vector derived from Agrobacterium (such as the aforementioned T-DNA vector), the plant is normally infected by introducing the vector into plant tissue by a physical method (see, for example, the aforementioned Non-Patent Document 1: Grimsley, N. et al.). Since vectors derived from Agrobacterium exhibit potent infectious capacity due to the action of the T-DNA domain, if infected by allowing to spread extensively throughout the entire plant by a physical method (such as injection or infiltration), a foreign gene can be uniformly expressed throughout all plant tissue. However, as was previously described, since the expression level of the foreign gene in each cell ends up being limited by the abilities of Agrobacterium regulatory sequences (such as nos promoter and nos terminator), it was difficult to improve expression level in the entire plant.

### [1-2. Cucumber Mosaic Virus]

Cucumber mosaic virus (to be suitably abbreviated as "CMV") is a virus belonging to the Bromoviridae family that is pathogenic to plants, and has a genome composed of three single-stranded RNA (RNA1, RNA2 and RNA3). RNA1, 2 and 3 have been developed into plasmids in the form of pCY1, pCY2 and pCY3, respectively (Suzuki, M., et al., Virology, Vol. 183, p. 106-113 (1991)).

RNA1 (pCY1) contains a gene that encodes 1a protein (replicase). RNA2 (pCY2) contains a gene that encodes 2a protein (RNA polymerase) and a gene that encodes 2b protein. RNA3 (pCY3) contains a gene that encodes 3a protein (intercellular transfer-associated protein) and a gene that encodes a membrane protein.

Furthermore, a plasmid vector (C2-H1) has been developed that enables recombination of a foreign gene by introducing a multicloning site into the gene of pCY2 that encodes 2b protein (Matsuo, K., et al., Planta, Vol. 225, p. 277-286 (2007)). Use of this plasmid makes it possible to transiently express a foreign gene in cells in plant.

According to techniques that use such CMV-derived vectors, the expression level of a foreign gene per cell in plant can be enhanced since the foreign gene is expressed using the high self-replication ability of CMV. However, since migration to each tissue and cell of a plant is dependent upon the migration ability of the virus, it is extremely difficult to express a foreign gene by infecting the cells of an entire plant with a virus. As a result, following vector introduction, the plant ends up in a chimeric (mosaic) state consisting of cells to which the virus has migrated that express the foreign gene and cells to which the virus has not migrated that do not express the foreign gene. Accordingly, it has remained difficult to improve expression level in an entire plant.

### [2. Nucleic Acid Molecule for Expressing a Foreign Gene in a Plant]

According to a first aspect of the present invention, a nucleic acid molecule is provided for expressing a foreign gene in a plant. (Furthermore, this nucleic acid molecule is subsequently suitably referred to as the "nucleic acid molecule of the present invention". In addition, a plant serving as the target of expression of a foreign gene using the nucleic acid molecule of the present invention is hereinafter suitably referred to as the "target plant".)

The nucleic acid molecule of the present invention has the following sequences (a) to (e) in the following order. However, sequence (d) may be optionally selected:
(a) a right border sequence (RB) derived from Agrobacterium T-DNA sequence;
(b) an expression promoter sequence that functions in a target plant;
(c) a sequence corresponding to the RNA2 genome of CMV in which a part or all of the gene encoding the 2b protein has been replaced with the foreign gene (to be subsequently suitably indicated as the "sequence corresponding to CMV RNA2 containing a target gene");
(d) a terminator sequence that functions in the target plant; and,
(e) a left border sequence (LB) derived from Agrobacterium T-DNA.

The aforementioned sequences (a) and (e), namely the right border sequence (RB) and the left border sequence (LB) derived from Agrobacterium T-DNA, are sequences involved in random incorporation of the Agrobacterium T-DNA sequence in a genome as previously described.

There are no limitations on the type of the aforementioned expression promoter sequence of (b) provided it is a sequence that functions in the genome of the target plant and initiates transcription of the coding sequence of the foreign gene. However, from the viewpoint of expressing the foreign gene in the target plant with high efficiency, a promoter is preferably used that possesses activity that is more potent than an nos promoter derived from Agrobacterium. Examples of such promoters include 35S RNA promoter of cauliflower mosaic virus (CaMV) (Odell, et al., Nature, Vol. 313, p. 810-812 (1985)), casaba mosaic virus promoter, figwort mosaic virus promoter, badnavirus promoter, strawberry vein banding virus (SVBV) promoter, mirabilis mosaic virus (MMV) promoter, rubisco promoter, actin promoter and ubiquitin promoter. Among these, a promoter derived from 35S RNA of cauliflower mosaic virus (CaMV) is preferable.

The cDNA sequence of the RNA2 genome is normally used for the sequence corresponding to the RNA2 genome of CMV in the sequence corresponding to the RNA2 genome containing a foreign gene of the aforementioned (c). As was previously described, the RNA2 genome of CMV contains a gene encoding 2a protein and a gene encoding 2b protein. In the present invention, a part or all of the sequence corresponding to the gene encoding the 2b protein in the cDNA sequence of the RNA2 genome is replaced with a foreign gene.

Although the type of replacement is arbitrary, from the viewpoint of improving transcription efficiency of the foreign gene, the start codon of the coding sequence of the foreign gene is preferably replaced starting downstream of the subgenomic promoter for expressing RNA2b. In addition, although the length of the foreign gene preferably coincides with the length of the sequence replaced in the gene encoding 2b protein so that the lengths of the sequences before and after replacement are the same, the length of the foreign gene may be longer or shorter than the length of the replaced sequence and it is not necessary to align the lengths before and after replacement provided incorporation of the RNA2 genome into CMV particles is not inhibited.

In addition, various modifications may be added to this sequence for the purpose of, for example, improving transcription/translation efficiency in a virus. Preferable examples of modifications include incorporation of a specific sequence near the start codon (AUG (such as "A/TXXAUGXC", where X represents an arbitrary base), incorporation of an internal ribosomal entry site (IRES) sequence, and incorporation of a sequence capable of being cleaved by ribosome.

The aforementioned terminator sequence of (d) is an optionally selected element as previously described. However, from the viewpoint of reliably terminating transcription of the coding sequence of the foreign gene and expressing a desired functional protein, the nucleic acid molecule of the present invention preferably has a terminator sequence. There are no limitations on the type of terminator sequence provided it is a sequence capable of terminating transcription of the coding sequence of the foreign gene. Examples thereof include Agrobacterium-derived nos terminator, heat shock protein (hsp) terminator and 35S RNA terminator of cauliflower mosaic virus (CaMV).

In addition, the expression promoter sequence of the aforementioned (b) and the sequence corresponding to CMV RNA2 containing a foreign protein of the aforementioned (c) (along with the optional sequence in the form of the terminator sequence of the aforementioned (d)) are operably linked so as to enable expression of a functional gene contained in (c) (namely, a gene encoding 2a protein and the foreign gene). Namely, the aforementioned sequences of (b) and (c) (and optionally sequence (d)) compose an expression cassette capable of autonomous expression in a plant genome. (Furthermore, this expression cassette is subsequently suitably referred to as the "expression cassette of the present invention".) In other words, the expression cassette of the present invention results in autonomous expression of 2a protein and foreign gene by being incorporated into a plant genome by random incorporation of the right border sequence (RB) and the left border sequence (LB).

The nucleic acid molecule of the present invention may also have other sequences provided they do not substantially impair the function thereof. Examples of other sequences include other arbitrary sequences derived from Agrobacterium Ti plasmid (such as a vir domain) and selection marker genes. Selection marker genes are used for the purpose of introducing the nucleic acid molecule of the present invention. Although there are no limitations on the type thereof, typical examples thereof include various types of antibiotic resistance genes and drug resistance genes, such as genes resulting in resistance to ampicillin, streptomycin, gentamycin, picromycin, actinonin (PDF1 gene), bialaphos herbicide, glyphosphate herbicide, sulfonamide or mannose. In addition, together with selection markers normally being operably linked with a unique regulatory sequence such as a promoter and composed in the form of an expression cassette capable of autonomic expression in a plant genome (to be subsequently suitably referred to as a "selection marker expression cassette"), they are arranged between the right border sequence (RB) of the aforementioned (a) and the left border sequence (LB) of the aforementioned (e) (on the 3' side or 5' side of the expression cassette of the present invention). As a result, the selection marker expression cassette is incorporated into a plant genome (along with the expression cassette of the present invention) resulting in autonomic expression as a result of random incorporation of the right border sequence (RB) and left border sequence (LB) into a genome.

The nucleic acid molecule of the present invention may have a linear form or cyclic form. However, it preferably is in a cyclic form such as in the form of a plasmid, and particularly is in the form of a T-DNA vector capable of replicating in Agrobacterium.

The nucleic acid molecule of the present invention having the aforementioned composition can easily be produced by suitably combining and using various types of gene recombination technologies known among persons with ordinary skill in the art.

The nucleic acid molecule of the present invention can be used to express a foreign gene in a target plant. The following provides a detailed explanation of the method thereof.

### [3. Method for Expressing a Foreign Gene in a Plant]

According to a second aspect of the present invention, a method is provided for expressing a foreign gene in a plant using the previously described nucleic acid molecule of the present invention. (Furthermore, this method is subsequently suitably referred to as the "method of the present invention".)

The method of the present invention at least comprises the following steps (i) to (iii):
(i) a step for respectively and functionally expressing the RNA1 genome of CMV, the RNA3 genome of CMV and the 2b protein of CMV in a target plant;
(ii) a step for introducing the nucleic acid molecule of the present invention into the target plant; and,
(iii) a step for cultivating the target plant introduced with the nucleic acid molecule of the present invention to express a foreign gene incorporated in the nucleic acid molecule of the present invention.

There are no particular limitations on the target plant used in the method of the present invention and any arbitrary species of plant can be used. In addition, tissue of a portion thereof as well as a culture of cells and the like can also be used in addition to the plant.

Examples of plants to which the present invention can be applied include alfalfa, barley, kidney beans, canola, cowpeas, cotton, corn, clover, lotus flower, lentil, lupine, sugar cane, oats, peas, peanuts, rice, rye, sweet clover, sunflower, sweet peas, soybeans, sorghum, triticale, yam beans, velvet beans, broad beans, wheat, wisteria and nut plants.

Preferable examples include gramineous plants, asteraceae plants, solanaceous plants and Rosaceae plants.

More preferable examples of plants include plants belonging to the following genii: Arabidopsis, Agrostis, Allium, Antirrhinum, Apium, Arachis, Asparagus, Atropa, Avena, Bambusa, Brassica, Bromus, Browallia, Camellia, Cannabis, Capsicum, Cicer, Chenopodium, Cichorium, Citrus, Coffea, Coix, Cucumis, Cucurbita, Cynodon, Dactylis, Datura, Dacus, Digitalis, Dioscorea, Elaeis, Eleusine, Festuca, Fragaria, Geranium, Glycine, Helianthus, Hemerocallis, Hevea, Hordeum, Hyoscyamus, Ipomoea, Lactuca, Lens, Lilium, Linum, Lolium, Nelumbo, Lycopersicon, Majorana, Malus, Mangifera, Manihot, Medicago, Nemesia, Nicotiana, Onobrychis, Oryza, Panicum, Pelargonium, Pennisetum, Petunia, Pisum, Phaseolus, Phleum, Poa, Prunus, Ranunculus, Raphanus, Ribes, Ricinus, Rubus, Saccharum, Salpiglossis, Secale, Senecio, Setaria, Sinapis, Solanum, Sorghum, Stenotaphrum, Theobroma, Trifolium, Trigonella, Triticum, Vicia, Vigna, Vitis and Zea.

There are also no particular limitations on the foreign gene used in the method of the present invention, and any arbitrary gene can be used. Examples thereof include naturally-occurring and synthetic genes along with fragments, mutants and variants thereof.

Specific examples of foreign genes include various types of cytokines, immunogenic substances, antibodies, enzymes, blood-derived components, adjuvant agonists, virus-derived components and pathogenic microorganism-derived components.

In step (i), the RNA1 genome of CMV, the RNA3 genome of CMV and 2b protein of CMV are respectively and functionally expressed in a target plant.

This step is achieved by, for example, introducing a sequence corresponding to the RNA1 genome of CMV, a sequence corresponding to a gene that encodes 2b protein of the RNA2 genome of CMV and a sequence corresponding to the RNA3 genome of CMV into a target plant and functionally expressing by incorporating into the plant genome. There are no limitations on the timing or order in which these sequences are introduced into the target plant. Namely, these sequences may be introduced simultaneously or may be introduced separately in any arbitrary order.

Furthermore, in step (i), additionally remaining sequences may be introduced as necessary using an existing plant such as a recombinant plant already having a part of all of these sequences followed by using them in the following step (ii). In addition, introduction of a part or all of these sequences in step (i) into a target plant may also be carried out simultaneous to introduction of the nucleic acid molecule of the present invention into the target plant in step (ii).

In step (i), a method based on transformation using a plant expression vector is normally used to introduce the sequence corresponding to the RNA1 genome of CMV, the sequence corresponding to a gene that encodes 2b protein of the RNA2 genome of CMV and the sequence corresponding to the RNA3 genome of CMV into a target plant.

Any arbitrary plant expression vector can be used for the plant expression vector. Examples thereof include various types of virus vectors, phage vectors and plasmid vectors. Preferable specific examples of virus vectors include vectors derived from various types of single-stranded RNA viruses such as tobacco mosaic virus (TMV), cucumber mosaic virus (CMV), cauliflower mosaic virus (CaMV), potato virus X (PVX) or clover yellow vein virus (CLYVV). A particularly preferable example thereof is a vector derived from cucumber mosaic virus (CMV). Examples of methods used to transform target cells in plant using these plant expression vectors include microinjection, electroporation, PEG-mediated protoplast transformation and particle gun method. The plant expression vector and specific transformation conditions may be suitably selected from among known conditions corresponding to the target plant, introduced sequences and the like.

In addition, the RNA1 genome of CMV, 2b protein of the RNA2 genome of CMV and the RNA3 genome of CMV can also be introduced and expressed in a target plant by respectively using the following nucleic acid molecules A, B and C having the same composition as the previously described nucleic acid molecule of the present invention.

Nucleic acid molecule A has the following sequences (a), (b), (c'), (d) and (e) in the order indicated below. However, sequence (d) may be optionally selected:
(a) right border sequence (RB) derived from Agrobacterium T-DNA sequence;
(b) expression promoter sequence that functions in the target plant;
(c') sequence corresponding to the RNA1 genome of CMV;
(d) terminator sequence that functions in the target plant; and,
(e) left border sequence (LB) derived from Agrobacterium T-DNA sequence.

Sequences (a), (b), (d) and (e) are as previously explained with respect to the nucleic acid molecule of the present invention.

The cDNA sequence of the RNA1 genome of CMV is normally used for sequence (c'), namely the sequence corresponding to the RNA1 genome of CMV. Various modifications may be applied to this sequence for the purpose of improving virus transcription/translation efficiency and the like. Examples of preferable modifications include the various types of modifications previously described with respect to sequence (c) of the nucleic acid molecule of the present invention.

In nucleic acid molecule A as well, the expression promoter sequence of the aforementioned (b) and the sequence corresponding to the RNA1 genome of CMV of the aforementioned (c') (and the optional sequence of the aforementioned (d) in the form of a terminator sequence) are operably linked so as to enable expression of the RNA1 genome of (c'), and compose an expression cassette that enables autonomous expression in a plant genome. (Furthermore, this expression cassette is subsequently suitably referred to as "expression cassette A".) This expression cassette A autonomously expresses the RNA1 genome by being incorporated into a plant genome by random incorporation of the right border sequence (RB) of (a) and the left border sequence (LB) of (e).

Nucleic acid molecule B has the following sequences (a), (b), (c''), (d) and (e) in the order indicated below. However, sequence (d) may be optionally selected:
(a) right border sequence (RB) derived from Agrobacterium T-DNA sequence;
(b) expression promoter sequence that functions in the target plant;
(c") sequence corresponding to the RNA3 genome of CMV;
(d) terminator sequence that functions in the target plant; and,
(e) left border sequence (LB) derived from Agrobacterium T-DNA sequence.

Sequences (a), (b), (d) and (e) are as previously explained with respect to the nucleic acid molecule of the present invention.

The cDNA sequence of the RNA2b of CMV is normally used for sequence (c"), namely the sequence corresponding to the 2b domain of the RNA2 genome of CMV. Various modifications may be applied to this sequence as well for the purpose of improving virus transcription/translation efficiency and the like. Examples of preferable modifications include the various types of modifications previously described with respect to sequence (c) of the nucleic acid molecule of the present invention.

In nucleic acid molecule B as well, the expression promoter sequence of the aforementioned (b) and the sequence corresponding to the RNA3 genome of CMV of the aforementioned (c") (and the optional sequence of the aforementioned (d) in the form of a terminator sequence) are operably linked so as to enable expression of 2b domain of the RNA2 genome of (c"), and compose an expression cassette that enables autonomous expression in a plant genome. (Furthermore, this expression cassette is subsequently suitably referred to as "expression cassette B".) This expression cassette B autonomously expresses the RNA3 genome by being incorporated into a plant genome by random incorporation of the right border sequence (RB) of (a) and the left border sequence (LB) of (e).

Nucleic acid molecule C has the following sequences (a), (b), (c"'), (d) and (e) in the order indicated below. However, sequence (d) may be optionally selected:
(a) right border sequence (RB) derived from Agrobacterium T-DNA sequence;
(b) expression promoter sequence that functions in the target plant;
(c''') sequence corresponding to the RNA3 genome of CMV;
(d) terminator sequence that functions in the target plant; and,
(e) left border sequence (LB) derived from Agrobacterium T-DNA sequence.

Sequences (a), (b), (d) and (e) are as previously explained with respect to the nucleic acid molecule of the present invention.

The cDNA sequence of the RNA3 genome of CMV is normally used for sequence (c'''), namely the sequence corresponding to the RNA3 genome of CMV. Various modifications may also be applied to this sequence for the purpose of improving virus transcription/translation efficiency and the like. Examples of preferable modifications include the various types of modifications previously described with respect to sequence (c) of the nucleic acid molecule of the present invention.

In nucleic acid molecule C as well, the expression promoter sequence of the aforementioned (b) and the sequence corresponding to the RNA3 genome of CMV of the aforementioned (c''') (and the optional sequence of the aforementioned (d) in the form of a terminator sequence) are operably linked so as to enable expression of the RNA3 genome of (c"'), and compose an expression cassette that enables autonomous expression in a plant genome. (Furthermore, this expression cassette is subsequently suitably referred to as "expression cassette C".) This expression cassette C autonomously expresses the RNA3 genome by being incorporated into a plant genome by random incorporation of the right border sequence (RB) of (a) and the left border sequence (LB) of (e).

Nucleic acid molecules A, B and C may also have other sequences provided they do not substantially impair the functions thereof. Examples of other sequences include other arbitrary sequences derived from Agrobacterium Ti plasmid (such as a vir domain) and selection marker genes. Selection marker genes are as previously described.

Nucleic acid molecules A, B and C may have a linear form or cyclic form. However, they are preferably in a cyclic form such as in the form of a plasmid, and particularly preferably are in the form of a T-DNA vector having the ability to replicate in Agrobacterium.

Nucleic acid molecules A and B exhibit potent infectious capacity against cells in plant due to the action of the right border sequence (RB) and left border sequence (LB) derived from Agrobacterium T-DNA sequence. Namely, a sequence that induces highly efficient random incorporation into the genome of cells in plant interposed between the right border sequence (RB) and the left border sequence (LB) (such as the RNA1 genome or RNA3 genome) is incorporated into a plant genome and is expressed in the plant. Accordingly, if infected by allowing to spread extensively throughout the entire plant by a physical method (such as injection or infiltration), a foreign gene can be uniformly expressed through the cells of all plant tissue.

In the case of introducing RNA1 genome and/or the 2b domain of the RNA2 genome and/or the RNA3 genome of CMV into a target plant using nucleic acid molecule A and/or nucleic acid molecule B and/or nucleic acid molecule C, although there are no limitations on the introduction method used, a solution containing these nucleic acid molecules is prepared and that solution is preferably introduced into plant tissue by various types of physical methods such as injection or infiltration. The same method and conditions as those used to introduce the nucleic acid molecule of the present invention into a target plant can be used for the specific conditions of the method used.

Furthermore, the aforementioned nucleic acid molecules A, B and C as well as various other vectors can be easily produced by suitably combining and using various types of gene recombination technologies known among persons with ordinary skill in the art.

In step (ii), the nucleic acid molecule of the present invention is introduced into a target plant.

Although there no limitations on the introduction method, a preferable example thereof consists of preparing a solution containing the nucleic acid molecule of the present invention and introducing that solution into plant tissue using various types of physical methods such as injection or infiltration.

A solution containing the nucleic acid molecule of the present invention can be prepared by culturing a microorganism such as Agrobacterium that produces the nucleic acid molecule of the present invention and then using the resulting culture (such as an overnight culture). Normally, an overnight culture reaches an optical density at a wavelength of 600 nm (OD₆₀₀) of 3 to 3.5 units. This culture is then diluted 3 to 5 times prior to agroinfiltration to typically form 5 × 10⁹ to 9 × 10⁹ colony forming units (Turpen, et al., J. Virol. Methods, Vol. 42, p. 227-240 (1993)). The resulting culture is then diluted 10²-fold, preferably 10³-fold and more preferably 10⁴-fold using a buffer such as MES buffer to suspend at an OD₆₀₀ value of 0.2 to 0.8 units. In the case of using each of these microbial solutions alone, suspensions are prepared at an OD₆₀₀ value of 0.2 to 0.6 units. In the case of using a mixture of a plurality of these microbial solutions, equal amounts of each of the bacterial cells are mixed followed by adjusting the final amount of the bacterial cells in the microbial solutions to an OD₆₀₀ value of 0.3 to 1.2 units.

In the case of introducing the nucleic acid molecule of the present invention into a target plant by injecting a solution thereof, introduction is carried out by forcibly injecting the solution into the target plant using a syringe and the like. In the case of introducing a solution containing the nucleic acid molecule of the present invention into a target plant by infiltration, introduction is carried out by first allowing the solution containing the nucleic acid molecule of the present invention to contact the target plant followed by forcibly causing the solution to infiltrate the target plant under reduced pressure conditions using, for example, a vacuum desiccator.

The nucleic acid molecule of the present invention is preferably allowed to uniformly spread throughout all tissue of the target plant by using the aforementioned techniques.

Furthermore, a vir domain derived from Ti plasmid as previously described is required to induce random incorporation of the right border sequence (RB) and left border sequence (LB) derived from the T-DNA sequence into a plant genome. Accordingly, in the case the nucleic acid molecule of the present invention does not have a vir domain, a helper plasmid having that vir domain is introduced together with the nucleic acid molecule of the present invention. On the other hand, a helper plasmid is not required in the case the nucleic acid molecule of the present invention has a vir domain.

In addition, in the case introduction of the RNA1 domain and/or RNA3 domain of CMV into a target plant is carried out using the aforementioned nucleic acid molecule A and/or nucleic acid molecule B in step (i), introduction may be carried out simultaneous to introduction of the nucleic acid molecule of the present invention into the target plant in step (ii). In this case, a solution containing the nucleic acid molecule of the present invention and a solution containing nucleic acid molecule A and/or nucleic acid molecule B are prepared, and those solutions are preferably simultaneously introduced into plant tissue by a single procedure such as injection or infiltration. Here, a nucleic acid molecule cassette composed of the nucleic acid molecule of the present invention and nucleic acid molecule A and/or nucleic acid molecule B, such as a Ti vector cassette, is also an object of the present invention.

In step (iii), the target plant introduced with the nucleic acid molecule of the present invention is cultivated to express a foreign gene incorporated in the nucleic acid molecule of the present invention. The method used to cultivate the target plant may be suitably selected corresponding to the type of target plant and purpose of expressing the foreign gene.

### [4. Summary]

The nucleic acid molecule of the present invention as explained above has potent infectious capacity against cells in plant due to the action of a right border sequence (RB) and left border sequence (LB) derived from Agrobacterium T-DNA sequence. Namely, a sequence that induces highly efficient random incorporation into the genome of cells in plant interposed between the right border sequence (RB) and the left border sequence (LB) (such as previously described expression cassette of the present invention) is incorporated into a plant genome and is expressed in the plant. Accordingly, according to the present invention, if infected by allowing to spread extensively throughout the entire plant by a physical method (such as injection or infiltration), a foreign gene can be uniformly expressed throughout the cells of all plant tissue.

In addition, the nucleic acid molecule of the present invention has a foreign gene within a gene that encodes 2b protein of the RNA2 genome of CMV, and is composed so as to autonomously express the foreign gene together with the 2a protein of CMV under the control of its own expression promoter sequence. Accordingly, by infecting a plant that respectively and functionally expresses the RNA1 genome of CMV, the RNA3 genome of CMV and the 2b protein of CMV with the nucleic acid of the present invention, the CMV genome expression product undergoes trans compensation, enabling the foreign gene to be expressed by using the high self-replication ability of CMV. In turn, the expression level of the foreign gene per cell in plant can be enhanced and the degree of freedom can be increased with respect to the type of host plant targeted by the nucleic acid molecule of the present invention.

Moreover, since the nucleic acid molecule of the present invention is able to minimize sequences other than that of a foreign gene to a greater degree than that of a conventional MagnICON^{®} system, it offers the advantage over the prior art of enabling the insertion of large foreign genes.

### [Examples]

The following provides a more detailed explanation of the present invention by indicating examples thereof. However, the present invention is not limited to the following examples, and can be worked by suitably modifying in various ways.

Furthermore, in the following description, detection of GFP fluorescence was carried out by acquiring fluorescence micrographs using a GFP epifluorescence illumination system (Model SZX-RFL2 SZX Fluorescence Illumination System, Olympus Corp.).

### [Experiment 1]

### Construction of Plant Expression Vector of CMV2b Protein

CMV2b gene was inserted downstream from an internal promoter sequence derived from a virus inserted with a plant expression promoter having a hygromycin resistance gene expression cassette (pGPTV-HPT). PRC was then carried out using the RNA2 genome of CMV as template and using CMV-2b-F1 (SEQ ID NO: 1: 5'-ATGGAATTGAACGTAGGTGCAATG-3') and CMV-2b-R1 (SEQ ID NO: 2: 5'-TCAGAAAGCACCTTCCGCC-3') designed on both ends of the 2b gene as primers to obtain an amplification product having a length of 333 bp. TA cloning was then carried out to obtain an XbaI-2b gene-SacI fragment imparted with a restrictase sequence. The plant expression vector pGPTV-HPT was linearized by cleaving with restrictases XbaI and SacI followed by ligation of the XbaI-2b gene-SacI fragment to construct pGPTV-HPT-2b (nucleic acid molecule B). Restrictase site confirmation and ligation were then carried out on the sequence of this cDNA clone using the ABI PRISM Big Dye Terminator (Applied Biosystems Inc. USA) to confirm the absence of sequence errors. The structure of the plant expression vector of the CMV2b gene is schematically shown in FIG. 1.

### [Experiment 2]

### Transformation of Agrobacterium with Plant Expression Vector

The freeze thaw method using liquid nitrogen (Holsters, M. et al., Mol. Gen. Genet., Vol. 163(2), p. 181-187 (1978)) was used to transform Agrobacterium (LBA4404) with plant binary vector. The resulting transformed Agrobacterium cells (LBA4404) were shake-cultured overnight at 28°C in LB medium containing 100 mg/l of rifampicin, 300 mg/l of streptomycin and 50 mg/l of kanamycin to obtain a cell liquid.

### [Experiment 3]

### Production of 2b Transgenic Plant

Transformation of tobacco (Nicotiana benthamiana) was carried out according to the leaf disk method (Horsch, R.B., et al., Science, Vol. 233, p. 496-498 (1984)) using Agrobacterium tumefaciens strain LBA4404. A leaf disk measuring about 1 cm in diameter was cut out from a tobacco leaf and immersed in a cell liquid of Agrobacterium tumefaciens strain LBA4404 retaining pGPTV-HPT-2b followed by co-culturing for 2 days on Murashige-Skoog (MS) agar medium. The co-cultured leaf disk was rinsed of Agrobacterium adhering thereto 3 days later followed by culturing (23°C, light/dark cycle: 16 hours of light, 8 hours of darkness) on MS redifferentiation medium containing 15 mg/l of hygromycin and 500 mg/l of carbenicillin and subculturing every 2 weeks. The resulting redifferentiated chutes were induced to take root in MS root formation medium containing 15 mg/l of hygromycin and 500 mg/l of carbenicillin followed by acclimating the cultured individuals to the recombination room temperature and growing by soil cultivation to obtain next-generation seeds (T1 individuals).

### [Experiment 4]

### 2b Protein Expression Analysis of 2b Transformant

After sampling leaves from the redifferentiated individuals (T0 individuals) and crushing using liquid nitrogen, the crushed leaves were ground after adding SDS-PAGE electrophoresis buffer. The resulting liquid was subjected to centrifugal separation (4°C, 1200 rpm, 10 minutes) and the supernatant was applied to SDS-PAGE. Western blotting analysis was then carried out with anti-2b antibody using a PVDF membrane for transfer. Transgenic tobacco that expressed 2b protein was then selected from among the redifferentiated individuals and used in the inoculation experiment of Experiment 6 to be subsequently described. A photograph showing the results of western blotting analysis is shown in FIG. 2.

### [Experiment 5]

### Construction of Binary Vector for Expressing Target Gene Inserted with CMV Genome Segments

Plasmids pBI-CR1, pBI-CR2 and pBI-CR3, in which full-length cDNA of genome segments of CMV was inserted into binary vector pBI121, were constructed in accordance with the method described in the doctoral thesis of Masashi Suzuki ("Research on the Functions of Cucumber Mosaic Virus", 1997, Tokyo University Doctoral Thesis, National Diet Library, Bibliographic ID: 000000343707). More specifically, cDNA was respectively amplified using the genome segments RNA1, RNA2 and RNA3 (virus genomes) possessed by CMV as templates, and each of the amplification products was incorporated between a CMV35S promoter (P35S) and nopaline synthase terminator (Tnos) followed by inserting into pBI121 to obtain plasmids pBI-CR1, pBI-CR2 and pBI-CR3. Moreover, in order to construct a vector enabling insertion of a target gene, the 2b gene domain of pBI-CR2 was replaced with jellyfish green fluorescent protein (GFP) gene, which is also used as a marker gene, to produce pBI-CR2Δ2b-GFP. First, using pCY2-H1-GFP, in which GFP gene was inserted between restrictase sites StuI and SpeI of pCY2-H1-GFP having for the backbone thereof a portion of CMV vector in the form of pUC118, as template, this vector was amplified by PCR using primers FW2a-Sal (SEQ ID NO: 3: 5'-TAGTTACGGTGTCGACACTCCC-3') and RVCS-pBi (SEQ ID NO: 4: 5'-GATCGGGGAAATTCGAGCTCACCGGGGTGGCGG-3') that amplify the region from the SalI restrictase site within the 2a gene of this vector to SacI on the 3'-terminal of the RNA2 virus genome. As a result, cDNA having a length of about 3290 bp was obtained. On the other hand, the previously constructed vector pBI-CR2 was linearized by digesting with restrictases SalI and SacI. After ligating the linearized vector and the roughly 3290 bp insert amplified by PCR using the In-Fusion Kit (Takara Bio Co.), the resulting ligation product was used to transform competent cells (HB101) and obtain pBI-CR2Δ2b-GFP clones. The sequence of these cDNA clones was confirmed for restrictase sites and confirmed to be free of sequencing errors. A vector containing CMV genome 2, in which the 2b gene domain had been replaced with a target gene in the form of GFP (nucleic acid molecule of the present invention), and a vector containing CMV genome 3 (nucleic acid molecule C), were produced according to the aforementioned procedure. Since the present invention is based on the use of three genome segments of CMV, it is necessary to simultaneously and in the same space express three virus genomes consisting of RNA1, RNA2 (inserted with target gene) and RNA3 within a plant. Consequently, vectors of these three molecular species are used as one set. The compositions of these binary vectors for plant expression are schematically shown in FIGS. 3(a) to 3(d). FIG. 3(a) shows a vector containing CMV genome 1 (nucleic acid molecule A), FIG. 3(b) shows a vector containing CMV genome 2, FIG. 3(c) shows a vector containing CMV genome 2 in which the 2b gene domain has been replaced with a target protein in the form of GFP (nucleic acid molecule of the present invention), and FIG. 3(d) shows a vector containing CMV genome 3 (nucleic acid molecule C).

### [Experiment 6]

### Agroinfiltration Method

A modified version of the method described in Grimsley, N. et al., Proc. Natl. Acad. Sci. USA, Vol. 83, p. 3282-3286 (1986) was used for the agroinfiltration method. Agrobacterium respectively retaining binary vectors inserted with genome segments of CMV and GFP gene in the form of pBI-CR1, pBI-CR2Δ2b-GFP and pBI-CR3 were transformed using the method described in Experiment 2 to obtain liquids of recombinant Agrobacterium individuals. Each liquid was centrifuged (20°C, 4800 rpm, 15 minutes) and the resulting cell pellets were suspended in MES buffer (final concentrations: MES 10 mM, MgCl₂ 10 mM, acetosyringone 150 µM, pH 5.7) to an OD₆₀₀ value of 0.3 to 1.2 units. In the case of using each of these cell solutions alone, suspensions are prepared to an OD₆₀₀ value of 0.2 to 0.6 units, while in the case of using by mixing a plurality of the cell solutions, equal amounts of each of the cells are mixed followed by adjusting the amount of cells in the suspension to an OD₆₀₀ value of 0.3 to 1.2 units. Nicotiana benthamiana was inoculated with the cell solutions by forced injection using a needle-free syringe or vacuum desiccator. A mixture of the Agrobacterium cells retaining 2b gene obtained using the methods of Experiment 1 and Experiment 2 was used in this procedure. Combinations of these inoculation vectors are shown in Table 1.

**[Table 1]**

| Table 1 Combinations of Inoculated Plants and Inoculated Agrobacterium Individuals (Retained Vectors) ("Yes": mixture of equal amounts, "-": unmixed individual) | | | | |
|---|---|---|---|---|
| Introduced Gene (Plasmid Name) | Sets of Mixed Individuals used in Agroinfiltration | | | |
| | 2b gene (pGPTV-HPT-2b) (nucleic acid molecule B, FIG. 1) | CMV-RNA1 (pBI-CR1) (nucleic acid molecule A, FIG. 3(a)) | CMV-RNA2 + target gene (pBI-CR2Δ2b-GFP) (nucleic acid molecule of present invention, FIG. 3(c)) | CMV-RNA3 (pBI-CR3) (nucleic acid molecule C, FIG. 3(d)) |
| Name of Inoculated Plant | | | | |
| Wild type plant | - | Yes | Yes | Yes |
| Wild type plant | Yes | Yes | Yes | Yes |
| 2b transgenic plant | - | Yes | Yes | Yes |

### [Experiment 7]

### Growth of Individual Inoculated Plants and Detection of Target Protein

Nicotiana benthamiana or recombinant plant bodies (transgenic plant bodies), inoculated with transgenic Agrobacterium by agroinfiltration, were cultivated in an environmentally-controlled room or illuminated plant growth incubator at 23°C using a light-dark cycle consisting of 16 hours of daylight and 8 hours of darkness. In order to confirm expression of the target gene (GFP) in the plant bodies following inoculation, the plant bodies were irradiated with blue light in the wavelength region that enables visualization of GFP followed by confirming fluorescence of GFP in the plant bodies after cultivating for 3 to 14 days. As a result of carrying out agroinfiltration on the combinations of inoculated plant bodies and inoculated Agrobacterium cells (retained vector) described in Table 1 using the method of Experiment 6, nearly all GFP was localized in the leaf veins in cases in which 2b protein or 2b gene was not present in the plant bodies. On the other hand, in the case of supplying 2b protein in the plant or transiently supplying 2b protein from Agrobacterium cells retaining 2b gene by agroinfiltration, GFP was able to be confirmed to be expressed throughout the entire leaf (leaf veins + leaf tissue)in the presence of 2b protein in all of the plant bodies.

Fluorescence micrographs indicating the results of detecting GFP in the leaves of the inoculated plant bodies are shown in FIGS. 4(A) and 4(B). FIG. 4(A) depicts fluorescence micrographs of leaves following agroinfiltration (5th day after inoculation) in wild type plant bodies and a 2b transgenic plant. FIG. 4(B) depicts fluorescence micrographs of the leaves of uninoculated plant bodies (negative controls). In FIGS. 4(A) and 4(B), GFP fluorescence micrographs were acquired using a GFP epifluorescence illumination system (Model SZX-RFL2 SZX Fluorescence Illumination System, Olympus Corp.). FIGS. 4(A) and 4(B) are photographs of fluorescence microscopic images of leaves in inoculated plant bodies.

### [Experiment 8]

### Production of Transgenic Plant Bodies Retaining CMV Genome Segment RNA1 or RNA3

In order to express a part (excluding 2a) or all of CMV protein in a plant, transgenic plant bodies retaining CMV genome segment RNA1 or RNA3 were first produced. Agrobacterium strain LBA4404, respectively retaining pBI-CR1 or pBI-CR3 constructed in Experiment 5, was transformed using the method described in Experiment 2 to obtain liquids containing recombinant Agrobacterium cells. Subsequently, Nicotiana benthamiana was transformed according to the leaf disk method (Horsch, R.B. et al., Science, Vol. 223, p. 496-498 (1984)) using the same procedure as that of Experiment 3 with the exception of changing the antibiotic from hygromycin to kanamycin. Leaf disks measuring about 1 cm in diameter were cut out from the tobacco leaves and immersed in a cell liquid of Agrobacterium tumefaciens strain LBA4404 retaining pBI-CR1 or pBI-CR3 followed by co-culturing for 2 days on Murashige-Skoog (MS) agar medium. The co-cultured leaf disks were rinsed of Agrobacterium adhering thereto 3 days later followed by culturing (23°C, light/dark cycle: 16 hours of light, 8 hours of darkness) on MS redifferentiation medium containing 50 mg/l of kanamycin and 500 mg/l of carbenicillin and subculturing every 2 weeks. The resulting redifferentiated chutes were induced to take root in MS root formation medium containing 50 mg/l of kanamycin and 500 mg/l of carbenicillin followed by acclimating the cultured individuals to the recombination room temperature and growing by soil cultivation to obtain next-generation seeds (T1 individuals).

### [Experiment 9]

### Functional Analysis of CMV Genome RNA1 or RNA3 Transformants as Plant Bodies for Inoculation

Next-generation seeds (T1 individuals) of CMV genome RNA1 or RNA3 transformants were aseptically inoculated into medium containing 125 mg/l of kanamycin followed by selecting those T1 individuals that were imparted with kanamycin resistance. The sprouts were grown indoors by cultivating in soil. After seeding, agroinfiltration was carried out on plant bodies that had been cultivated for about two months using the method described in Experiment 6. T1 individuals of CMV genome RNA1 transformants were prepared so that the OD₆₀₀ value of the final concentration of the amount of cells in a suspension obtained by mixing equal amounts of two types of Agrobacterium cell solutions having pBI-CR2Δ2b-GFP and pBI-CR3 at a 1:1 ratio was 0.6 units. In addition, T1 individuals of CMV genome RNA3 transformants were prepared so that the OD₆₀₀ value of the final concentration of the amount of cells in a suspension obtained by mixing equal amounts of two types of Agrobacterium cell solutions having pBI-CR1 and pBI-CR2Δ2b-GFP at a 1:1 ratio was 0.6 units. In both cases, inoculation of the recombinant plant bodies was carried out by forced injection using a needle-free syringe or vacuum desiccator. Moreover, transient expression was carried out by agroinfiltration in order to supply these transgenic plant bodies with the 2b gene required by the present invention. T1 individuals of CMV genome RNA1 transformants (CR1-Tg) were prepared so that the OD₆₀₀ value of the final concentration of the amount of cells in a suspension obtained by mixing equal amounts of three types of Agrobacterium cell solutions having pBI-CR2Δ2b-GFP, pB-CR3 and pGPTV-HPT-2b at a 1:1:1 ratio was 0.9 units. In addition, T1 individuals of CMV genome RNA3 transformants (CR3-Tg) were prepared so that the OD₆₀₀ value of the final concentration of the amount of cells in a suspension obtained by mixing equal amounts of three types of Agrobacterium cell solutions having pBI-CR1, pBI-CR2Δ2b-GFP and pGPTV-HPT-2b at a 1:1:1 ratio was 0.9 units, followed by forced injection into plant bodies. Following inoculation by agroinfiltration, the plant bodies were cultivated for 5 to 7 days according to the method described in Experiment 7.

Continuing, agroinfiltration was carried out with the combinations of inoculated plant bodies and inoculated Agrobacterium cells (retained vector) described in the following Table 2.

**[Table 2]**

| Table 2 Combinations of Inoculated Plants and Inoculated Agrobacterium Individuals (Retained Vectors) ("Yes": mixture of equal amounts,"-": unmixed individual) | | | | |
|---|---|---|---|---|
| Introduced Gene (Plasmid Name) | Sets of Mixed Individuals used in Agroinfiltration | | | |
| | 2b gene (pGPTV-HPT-2b) (nucleic acid molecule B, FIG. 1) | CMV-RNA1 (pBI-CR1) (nucleic acid molecule A, FIG. 3(a)) | CMV-RNA2 + target gene (pBI-CR2Δ2b-GFP) (nucleic acid molecule of present invention, FIG. 3(c)) | CMV-RNA3 (pBI-CR3) (nucleic acid molecule C, FIG. 3(d)) |
| Name of Inoculated Plant | | | | |
| CR1 transgenic plant | - | - | Yes | Yes |
| CR1 transgenic plant | Yes | - | Yes | Yes |
| CR3 transgenic plant | - | Yes | Yes | - |
| CR3 transgenic plant | Yes | Yes | Yes | - |

Nearly all GFP was localized in leaf veins in cases in which 2b protein or 2b gene was not present in the plant bodies. On the other hand, in the case of transiently supplying 2b from Agrobacterium cells retaining 2b gene, GFP was able to be confirmed to be expressed throughout the entire leaf (leaf veins + leaf tissue)in the presence of 2b protein in all of the plant bodies. The results of GFP detection in the inoculated plant bodies are shown in FIG. 5.

### [Experiment 10]

### Production of Plant Bodies for Inoculation from Transformants having CMV Genome RNA1 + 2b Gene or CMV Genome RNA3 + 2b Gene

Since GFP of a target gene was able to be confirmed in Experiment 7 to be expressed throughout the entire leaf (leaf veins + leaf tissue) in the presence of CMV 2b protein, T1 individuals of the transformants produced using the method described in Experiment 8 were grown at room temperature and the pistils of pre-pollenated flowers were artificially pollenated with the 2b transformant produced in Experiment 3 in order to produce transformants (Nicotiana benthamiana) that express a gene encoding this 2b protein along with CMV genome RNA1 or RNA3. The resulting seeds were aseptically seeded in MS medium containing 125 mg/l of kanamycin and 15 mg/l of hygromycin, and after selecting those plant bodies imparted with resistance to both kanamycin and hygromycin, the sprouts were transplanted and grown indoors by cultivating in soil. Genomic DNA was extracted from the leaves of these individuals followed by carrying out genomic PCR analysis, and the CR1 + 2b transformants were confirmed to be transgenic plant bodies having both pBI-CR1 and 2b gene, while the CR3 + 2b transformants were confirmed to be transgenic plant bodies having both pBI-CR3 and 2b gene.

### [Experiment 11]

### Functional Analysis of Transformants having CMV Genome RNA1 + 2b Gene or CMV Genome RNA3 + 2b Gene as Plant Bodies for Inoculation

After aseptically inoculating into MS medium containing 125 mg/l of kanamycin and 15 mg/l of hygromycin and selecting plant bodies imparted with both kanamycin and hygromycin resistance, the sprouts were grown indoors by cultivating in soil. After seeding, agroinfiltration was carried out on plant bodies that were cultivated for about 2 months using the method described in Experiment 6. CR1 + 2b transformants were prepared so that the OD₆₀₀ value of the final concentration of the amount of cells in a suspension obtained by mixing equal amounts of two types of Agrobacterium cell solutions having pBI-CR2Δ2b-GFP and pBI-CR3 at a 1:1 ratio was 0.6 units. In addition, T1 individuals of CR3 + 2b transformants were prepared so that the OD₆₀₀ value of the final concentration of the amount of cells in a suspension obtained by mixing equal amounts of two types of Agrobacterium cell solutions having pBI-CR1 and pBI-CR2Δ2b-GFP at a 1:1 ratio was 0.6 units. In both cases, inoculation of the recombinant plant bodies was carried out by forced injection using a needle-free syringe or vacuum desiccator. Following inoculation by agroinfiltration, the plant bodies were cultivated for 5 to 7 days according to the method described in Experiment 7. The results are shown in the following Table 3.

**[Table 3]**

| Table 3 Combinations of Inoculated Plants and Inoculated Agrobacterium Individuals (Retained Vectors) ("Yes": mixture of equal amounts, "-": unmixed individual) | | |
|---|---|---|
| Introduced Gene (Plasmid Name) | Sets of Mixed Individuals used in Agroinfiltration | |
| | Target gene + CMV-RNA3 (pBI-CR2Δ2b-GFP, pBI-CR3) (nucleic acid molecule of present invention, FIG. 3(c) and nucleic acid molecule C (FIG. 3(d)) | Target gene + CMV-RNA1 (pBI-CR2Δ2b-GFP, pBI-CR1) (nucleic acid molecule of present invention, FIG. 3(c) and nucleic acid molecule A (FIG. 3(a)) |
| Name of Inoculated Plant | | |
| CR1 transgenic plant | Yes | - |
| CR3 transgenic plant | - | Yes |

Fluorescence micrographs indicating the results of detecting GFP in the leaves of the inoculated plant bodies are shown in FIG. 6. FIG. 6 respectively depicts fluorescence micrographs of a leaf following agroinfiltration (5th day after inoculation) in a CR1 + 2b transformant (left side) and CR3 + 2b transformation (right side). GFP fluorescence micrographs were acquired in both cases using a GFP epifluorescence illumination system (Model SZX-RFL2 SZX Fluorescence Illumination System, Olympus Corp.). Expression of GFP fluorescence was clearly determined to be occurring in leaf tissue and leaf veins in both transformants.

### INDUSTRIAL APPLICABILITY

As explained above, the method according to the present invention employing a specific gene expression vector in combination with a transgenic plant allows for systemic transition of the vector into cells throughout the plant to cause infection, whereby high level expression of the target gene is achieved. In addition, transient expression can be achieved using various host plants and various target genes different in size. Accordingly, the present invention is highly applicable in the industrial fields of, e.g., agriculture, agricultural production engineering, resource production science, and biological production engineering.

## Claims

1. A nucleic acid molecule for expressing a foreign gene in a plant, comprising, arranged in this order:
a right border sequence (RB) derived from the *Agrobacterium* T-DNA sequence;
an expression promoter sequence workable in the plant;
a sequence corresponding to the RNA2 genome of cucumber mosaic virus in which a part or all of the gene coding for the 2b protein has been replaced with the foreign gene; and
a left border sequence (LB) derived from the *Agrobacterium* T-DNA sequence.

2. The nucleic acid molecule according to claim 1, further comprising a terminator sequence workable in the plant, arranged between the sequence corresponding to the RNA2 genome of cucumber mosaic virus and the left border sequence (LB) derived from Agrobacterium T-DNA sequence.

3. The nucleic acid molecule according to claim 1 or 2, which is in the form of a T-DNA vector.

4. The nucleic acid molecule according to any one of claims 1 to 3, wherein the plant functionally expresses the RNA1 genome of cucumber mosaic virus, the RNA3 genome of cucumber mosaic virus, and the 2b protein of cucumber mosaic virus.

5. A method for expressing a foreign gene in a plant, comprising:
functionally expressing the RNA1 genome of cucumber mosaic virus, the RNA3 genome of cucumber mosaic virus, and the 2b protein of cucumber mosaic virus in the plant;
introducing the nucleic acid molecule according to any one of claims 1 to 3 into the plant; and
culturing the plant containing the nucleic acid molecule to thereby express the foreign gene incorporated in the nucleic acid molecule in the plant.

6. The method according to claim 5, wherein the expression of the RNA1 genome of cucumber mosaic virus and the RNA3 genome of cucumber mosaic virus is carried out by introducing, into the plant:
a nucleic acid molecule comprising, arranged in this order, a right border sequence (RB) derived from *Agrobacterium* T-DNA sequence, an expression promoter sequence workable in the plant, a sequence corresponding to the RNA1 genome of cucumber mosaic virus, and a left border sequence (LB) derived from *Agrobacterium* T-DNA sequence; and
a nucleic acid molecule comprising, arranged in this order, a right border sequence (RB) derived from *Agrobacterium* T-DNA sequence, an expression promoter sequence workable in the plant, a sequence corresponding to the RNA3 genome of cucumber mosaic virus, and a left border sequence (LB) derived from *Agrobacterium* T-DNA sequence.

7. The method according to claim 5, wherein the introduction of the nucleic acid molecules into the plant is carried out by infiltrating or injecting a solution containing the nucleic acid molecules into the plant.


### SEQUENCE LISTING

<110> The National Institute of Advanced Industrial Science and Technology
<120> NUCLEIC ACID MOLECULES AND METHOD FOR MAKING PLANT EXPRESS FOREIGN
   GENE
<130> AC540-PCT
<150> JP2013-031355
   <151> 2013-02-20
<160> 4
<170> PatentIn version 3.4
<210> 1
   <211> 24
   <212> DNA
   <213> Artificial
<220>
   <223> sequence of forward primer CMV-2b-F1
<400> 1
   atggaattga acgtaggtgc aatg 24
<210> 2
   <211> 19
   <212> DNA
   <213> Artificial
<220>
   <223> sequence of reverse primer CMV-2b-R1
<400> 2
   tcagaaagca ccttccgcc 19
<210> 3
   <211> 22
   <212> DNA
   <213> Artificial
<220>
   <223> sequence of forward primer FW2a-Sal
<400> 3
   tagttacggt gtcgacactc cc 22
<210> 4
   <211> 33
   <212> DNA
   <213> Artificial
<220>
   <223> sequence of reverse primer RVCS-pBI
<400> 4
   gatcggggaa attcgagctc accgcggtgg cgg 33